# FASCICULE DE BREVET EUROPEEN

(11) **EP 2 694 032 B1**
(45) Date de publication et mention de la délivrance du brevet: **03.10.2018**
(21) Numéro de dépôt: 12720603.5
(22) Date de dépôt: 05.04.2012
(51) Int. Cl.: A61K 8/99, A61Q 5/00

(54) **NOUVEL ACTIF UTILE POUR PREVENIR ET/OU TRAITER LES ETATS PELLICULAIRES DU CUIR CHEVELU**
NEUER NÜTZLICHER WIRKSTOFF ZUR PRÄVENTION UND/ODER BEHANDLUNG VON SCHUPPEN AUF DER KOPFHAUT
NOVEL USEFUL ACTIVE AGENT FOR PREVENTING AND/OR TREATING DANDRUFF ON THE SCALP

(30) Priorité: 05.04.2011 FR 1101029
(43) Date de publication de la demande: 12.02.2014
(73) Titulaire: L'Oréal, 75008 Paris (FR)
(72) Inventeur: HILAIRE, Pascal, F-37210 Vouvray (FR); MAHE, Yann, 91700 Sainte Genevieve des Bois (FR); MARTIN, Richard, 37210 Rochecorbon (FR)
(74) Mandataire: Nony
(86) Numéro de dépôt international: PCT/IB2012/051698
(87) Numéro de publication internationale: WO 2012/137170

(56) Documents cités:
- EP-A1- 2 149 368
- EP-A1- 2 830 588
- EP-A2- 1 400 237
- FR-A1- 2 879 452
- FR-A1- 2 930 443

## Description

L'invention concerne l'utilisation cosmétique d'un actif dérivé d'un microorganisme appartenant au genre *Vitreoscilla sp.* (Notamment espèce: *Vitreoscilla filiformis)* à titre d'actif pour prévenir et/ou traiter les états pelliculaires du cuir chevelu, y compris des états pelliculaires associés à une prolifération de microorganismes pathogènes sur le cuir chevelu et/ou un déséquilibre de l'écoflore du cuir chevelu.

La peau est un tissu dont les cellules sont jointives, et solidaires les unes des autres. Le tissu cutané forme un revêtement externe comprenant des glandes sébacées ou sudoripares, et les follicules pileux. La peau, et notamment le cuir chevelu, sont des épithéliums à renouvellement continuel. Le renouvellement, ou desquamation, est un processus coordonné et finement régulé aboutissant à l'élimination des cellules superficielles, de façon insensible et non visible.

Toutefois, une desquamation anormale ou irrégulière des cellules du *stratum corneum,* pour diverses raisons, peut conduire à la formation d'amas de cellules de grandes tailles, épais, visibles à l'oeil nu, et dénommés « squames », ou « pellicules » dans le cadre du cuir chevelu, ou dans d'autres situations, à un amincissement du *stratum corneum.* Les troubles de la desquamation, résultant d'une desquamation anormale ou irrégulière, peuvent aboutir à une fragilité, voire un défaut des propriétés barrières de l'épiderme.

A titre d'exemple de facteurs favorisant l'apparition de squames ou de pellicules, on peut mentionner le stress, la période hivernale, un excès de sébum, un défaut d'hydratation, ou la colonisation de la peau ou des follicules pileux par la levure *Malassezia sp.* Ces facteurs ont, notamment, pour caractère commun de provoquer et/ou favoriser un état inflammatoire cutané. Une telle inflammation renforce l'apparition, voire augmente la présence de squames ou de pellicules. En particulier, les levures de type *Malassezia sp.*, constituant une partie de la flore commensale normale à la surface du cuir chevelu chez des sujets sans pellicule, voient leur proportion croître substantiellement en cas de pellicules, ou en cas de dermite séborrhéique associée. Le déséquilibre de l'écoflore du cuir chevelu est un facteur favorisant, voire renforçant la présence de pellicules.

La présence de squames ou les états pelliculaires peuvent être des états chroniques, fréquents, récidivants, et socialement invalidants du fait de leur caractère inesthétique manifeste. Qui plus est, les états pelliculaires du cuir chevelu ou la desquamation anormale de la peau peuvent se traduire par une altération de la fonction barrière de l'épiderme, ou générer des sensations de démangeaison ou prurit, conduisant à des comportements de grattage amplifiant le phénomène d'apparition de squames ou de pellicules, et, en retour, l'irritation du cuir chevelu ou de la peau.

Les états pelliculaires du cuir chevelu peuvent être de type gras ou de type sec. Les états pelliculaires secs du cuir chevelu se manifestent plus fréquemment, et sont amplifiés, lors de troubles de l'hydratation de la peau, et notamment lors de sécheresse importante de l'épiderme du cuir chevelu. Egalement, le cuir chevelu étant riche en glandes sébacées, un état pelliculaire peut se développer plus facilement en présence excessive de sébum et être plus facilement prurigineux. Ainsi, une sécrétion excessive de sébum, ou hyperséborrhée, favorise l'apparition d'un état pelliculaire gras du cuir chevelu, ou pellicules grasses, généralement associé à des désagréments, des sensations d'inconfort, des désordres esthétiques, voire une pathologie cutanée.

Les états pelliculaires répondent, en général, à différents traitements locaux ou systémiques. Cependant, l'efficacité de ces traitements n'est que suspensive et implique un suivi rigoureux de la part de l'utilisateur (fréquence d'utilisation et temps d'application suffisant). Or, un usage quotidien et à long terme de ces traitements peut entraîner un phénomène d'accoutumance réduisant leur efficacité, l'accoutumance étant généralement associée à un phénomène de rebond survenant à la cessation du traitement. Ce phénomène se manifeste par une hyperséborrhée, aggravant l'état pelliculaire et altérant la fonction barrière du cuir chevelu. Par ailleurs, l'agressivité de certains actifs antipelliculaires vis-à-vis des cellules épidermiques ou de l'écoflore du cuir chevelu peut également affecter les fonctions barrières de ce dernier et provoquer une aggravation de l'état pelliculaire. Enfin, l'efficacité des traitements antipelliculaires est souvent lente à se développer et nécessite une application rigoureuse sur le long terme. Ce temps de latence conduit fréquemment à un défaut du suivi du traitement. En conséquence, de nombreux échecs surviennent dans la mise en oeuvre de ces traitements.

De nombreux facteurs épidermiques, dont l'expression, l'activité biologique ou la maturation sont altérées, diminuées ou augmentées, sont connus pour être impliqués, directement ou indirectement, dans le processus de renouvellement, ou de desquamation du cuir chevelu.

Ces facteurs peuvent être utilisés à titre de biomarqueurs du cuir chevelu, comme cibles de criblage, voire comme actifs cosmétiques.

Toutefois, il demeure encore de nombreuses inconnues quant au mécanisme intime et aux facteurs impliqués dans la desquamation de la peau, et en particulier dans l'apparition des pellicules.

EP 2 149 368 décrit l'utilisation d'un lysat de *Vitreoscilla filiformis* comme agent antipelliculaire. Mais il existe encore un besoin de disposer de nouveaux actifs ou de nouveaux traitements pour prévenir et/ou traiter un trouble de la desquamation de la peau, et plus particulièrement un état pelliculaire du cuir chevelu. Il demeure également un besoin de disposer de nouveaux traitements cosmétiques pour prévenir, réduire et/ou traiter les états pelliculaires du cuir chevelu qui soient efficaces et dépourvus d'effets secondaires susceptibles d'affecter une bonne observance.

Il existe encore un besoin de disposer d'un traitement cosmétique des états pelliculaires du cuir chevelu n'affectant pas l'écoflore du cuir chevelu, voire renforçant la présence d'une écoflore saine.

Il existe un besoin de disposer de traitements cosmétiques des états pelliculaires apte à maintenir, voire à renforcer, les propriétés barrières du cuir chevelu.

Il existe un besoin de disposer de traitements cosmétiques des états pelliculaires qui soient dépourvus des effets secondaires précités, et en particulier qui n'induisent pas d'hyperséborrhées, de dermites séborrhéiques ou d'états prurigineux.

Il existe également un besoin de disposer d'un traitement cosmétique des états pelliculaires qui n'induise pas d'états inflammatoires.

La présente invention a pour objet de satisfaire à ces besoins.

Ainsi, la présente invention vise en particulier à proposer un nouvel actif répondant aux exigences précitées et manifestant en particulier une efficacité à l'égard des désordres de la fonction barrière du cuir chevelu face à une microflore pathogène. Comme cela est décrit en détail ci-après, le nouvel actif de l'invention est avantageusement utilisé dans la mise en oeuvre de procédés de traitement cosmétiques d'états pelliculaires du cuir chevelu.

Plus précisément, la présente invention concerne l'utilisation cosmétique d'un lysat de bactérie(s) appartenant au genre *Vitreoscilla sp.* (Notamment espece: *Vitreoscilla filiformis),* dans un milieu de fermentation complet, à titre d'actif pour prévenir et/ou traiter les états pelliculaires du cuir chevelu notamment associés à un déséquilibre de l'écoflore du cuir chevelu.

La prévention et/ou le traitement des états pelliculaires englobent la prévention et/ou le traitement des pellicules et/ou la prévention et/ou le traitement des démangeaisons du cuir chevelu liées à la présence de pellicules.

Au sens de la présente invention, l'expression « lysat dans un milieu de fermentation complet » signifie que le lysat est mis en oeuvre et est présent, dans la composition cosmétique ou dermatologique le contenant, formulé dans son milieu de culture complet d'origine tel que défini ci-après, lequel milieu de culture complet est le milieu dans lequel les bactéries ont été cultivées jusqu'après la phase de croissance microbienne ayant conduit à l'utilisation des substrats nutritifs présents initialement dans le milieu de culture.

Au sens de la présente invention, l'expression « milieu de fermentation complet » entend désigner un milieu issu du procédé de culture ayant été utilisé pour la croissance et la lyse cellulaire du microorganisme, ledit milieu n'ayant subi par ailleurs aucune manipulation additionnelle visant à séparer et/ou éliminer tout ou partie de ses constituants non aqueux.

Au sens de la présente invention, on entend par « prévenir » le fait de supprimer totalement ou de diminuer partiellement le risque de manifestation d'un phénomène donné, c'est-à-dire dans la présente invention la manifestation d'états pelliculaires du cuir chevelu. La diminution partielle implique que le risque demeure mais à un degré moindre qu'avant la mise en oeuvre de l'invention.

Une composition contenant l'actif selon l'invention peut être administrée par voie topique.

L'application d'un extrait de bactéries filamenteuses non fructifiantes non photosynthétiques à l'image des bactéries du genre *Vitreoscilla sp.* (Notamment espece *Vitreoscilla filiformis*) comme actif pour moduler et de préférence inhiber l'adhésion et/ou la prolifération de micro-organismes pathogènes sur la peau et le cuir chevelu a déjà été proposée dans le document FR 2 879 452.

Toutefois, l'extrait considéré dans ce document consiste en soit le surnageant du milieu de fermentation de ladite bactérie, soit la biomasse obtenue après culture des dites bactéries, soit les enveloppes ou fractions d'enveloppes ou encore extraits obtenus suite à un traitement complémentaire de la biomasse. En conséquence, l'ensemble des extraits précités n'est obtenu qu'à l'issue d'une opération complémentaire telle que par exemple filtration ou centrifugation, imposée au milieu de fermentation en vu de séparer l'extrait considéré des autres constituants de ce milieu de fermentation.

L'actif considéré selon l'invention est donc totalement distinct de l'extrait considéré et décrit dans le document FR 2 879 452.

Des compositions cosmétiques et/ou dermatologiques comprenant un extrait total de *Vitreoscilla filiformis* ont été décrites dans la demande de brevet européen n° EP 1 400 237. La demande EP 1 400 237 décrit divers extraits de *Vitreoscilla filiformis,* respectivement (i) des cellules bactériennes séparées de la biomasse, par exemple par centrifugation, (ii) la biomasse, laquelle est une suspension acellulaire susceptible de contenir des débris cellulaires et (iii) la fraction surnageante de cette biomasse.

Par opposition à l'extrait décrit dans FR 2 879 452 ou encore à ceux décrits dans EP 1 400 237, l'actif considéré selon l'invention est constitué de l'ensemble des composants présents dans le milieu de fermentation. L'effet surprenant de l'utilisation selon l'invention résulte donc de la mise en oeuvre, à titre d'actif, pour la première fois par les inventeurs d'un lysat de microorganisme dans son milieu de fermentation complet.

En effet et comme il ressort des exemples ci-après, les inventeurs ont constaté que l'actif conforme à l'invention manifeste une activité régulatrice de la microflore du cuir chevelu, supérieure à celle constatée pour un extrait de type biomasse de la même bactérie. Notamment, les inventeurs ont observé qu'à l'issu d'un traitement de l'invention, l'équilibre de la microflore et les propriétés barrières du cuir chevelu étaient renforcées.

Sans vouloir être lié par ce qui suit, ce gain d'efficacité pourrait être le résultat d'un effet synergique entre des constituants de la bactérie, d'ordinaire séparés les uns des autres, par exemple ses métabolites hydrosolubles, générés lors de sa prolifération dans son milieu de fermentation et conventionnellement présents dans le surnageant aqueux et ses composants telles que enveloppes ou fractions d'enveloppes cellulaires non hydrosolubles constituant tout ou partie de la biomasse de son milieu de culture voire son lysat isolé.

On a ainsi montré dans les exemples qu'une composition cosmétique comprenant un actif selon l'invention (un lysat de bactéries appartenant au genre *Vitreoscilla sp.* dans un milieu de fermentation complet) a des propriétés de réduction des états pelliculaires du cuir chevelu égales ou même supérieures à celles d'actifs antipelliculaires connus, tels que l'octopyrox ou le zinc pyrythione (ZnPT). En particulier, on a montré dans les exemples qu'une formulation cosmétique comprenant un actif de l'invention était actif contre les états pelliculaires du cuir chevelu à des doses beaucoup plus faibles que les doses d'actifs antipelliculaires conventionnels nécessaires à l'obtention des mêmes effets sur les pellicules du cuir chevelu.

On a aussi montré qu'un actif selon l'invention possède aussi des propriétés de réduction des démangeaisons du cuir chevelu provoquées par les états pelliculaires.

Contre toute attente, l'actif considéré selon l'invention semble en revanche dénué d'activité inhibitrice à l'égard des différentes souches de *malassezia sp* étudiées : *globosa* et *restricta,* comme cela est montré dans les exemples.

Selon un autre mode de réalisation, la présente invention concerne un procédé cosmétique pour prévenir et/ou traiter les états pelliculaires du cuir chevelu, y compris les états pelliculaires du cuir chevelu associés à une prévalence des microorganismes pathogènes sur le cuir chevelu et/ou un déséquilibre de l'écoflore du cuir chevelu chez un individu, comprenant au moins une étape d'administration audit individu, notamment par voie orale, une quantité efficace d'au moins un lysat de bactérie(s) appartenant au genre *Vitreoscilla sp.* (Notamment espèce: *Vitreoscilla filiformis*) dans un milieu de fermentation complet.

Au sens de la présente invention, on entend par « quantité efficace », une quantité suffisante et nécessaire du composé considéré pour obtenir l'effet attendu. Une telle quantité peut être déterminée par toute méthode connue de l'homme de l'art, par exemple au moyen d'essais expérimentaux préliminaires.

Selon un autre de ses aspects, la présente invention concerne une composition cosmétique et/ou dermatologique rincée ou non-rincée contenant dans un milieu physiologiquement acceptable au moins un lysat de microorganisme(s) appartenant au genre *Vitreoscilla sp.* (Notamment espèce: *Vitreoscilla filiformis),* dans un milieu de fermentation complet.

### Milieu de fermentation complet

Comme énoncé précédemment, l'expression « milieu de fermentation complet » entend désigner un milieu de fermentation ou encore de culture possédant la même composition, au moins en termes de constituants non aqueux voire dans sa totalité, que le milieu de fermentation dans lequel il a été procédé successivement à la fermentation et à la lyse cellulaire du microorganisme dédié à former le lysat requis parallèlement selon l'invention.

En d'autres termes, ce milieu n'a subi par ailleurs aucune manipulation annexe visant à séparer et/ou éliminer tout ou partie de ses constituants non aqueux.

L' actif considéré selon l'invention est formé du lysat de microorganismes et de tout ou partie, en terme de quantité, du milieu de culture ayant servi à la fermentation de ladite bactérie et dans lequel il a été procédé consécutivement à sa lyse cellulaire (i.e. milieu de fermentation complet).

Au regard de ce qui précède, il ressort que l'actif formé selon l'invention à partir du lysat et du milieu de fermentation dit complet contient les fractions cytoplasmique et cytosolique, les fragments de paroi cellulaire et les métabolites formés et/ou libérés lors de la lyse cellulaire dudit microorganisme, l'intégralité des entités biologiques susceptibles d'être générées et libérées spontanément par la bactérie lors de son processus de fermentation et donc déjà présentes dans le milieu de fermentation avant la lyse cellulaire de celle-ci.

En conséquence, un actif selon l'invention c'est-à-dire formé d'un lysat de bactérie appartenant au genre *Vitreoscilla sp.* (notamment espèce: *Vitreoscilla filiformis*) dans un milieu de fermentation complet selon l'invention est clairement différent du surnageant d'un milieu de fermentation d'une bactérie appartenant au genre *Vitreoscilla sp.* (Notamment espèce: *Vitreoscilla filiformis*)*.*

En effet, l'actif considéré selon l'invention, par opposition au surnageant, contient des fragments cellulaires de ladite bactérie figurés par le lysat.

Un actif selon l'invention c'est-à-dire formé d'un lysat de bactérie appartenant au genre *Vitreoscilla sp.* (Notamment espèce: *Vitreoscilla filiformis)* dans un milieu de fermentation complet selon l'invention est également différent de la biomasse ou fraction de biomasse, voire d'un lysat ou fraction de lysat isolé d'un milieu de fermentation d'une bactérie appartenant au genre *Vitreoscilla sp.* (Notamment espèce: *Vitreoscilla filiformis).*

En effet l'actif considéré selon l'invention par opposition à cette biomasse, ou fraction de biomasse, ce lysat ou fraction de lysat contient une quantité significative de métabolites hydrosolubles libérés naturellement dans le milieu de culture lors de la prolifération de ladite bactérie.

Au sens de la présente invention, l'expression « constituants non aqueux » sous entend que l'eau, qui est un constituant majoritaire des milieux de fermentation conventionnels ne fait pas partie des constituants devant demeurer en tant que tels, c'est-à-dire à quantité égale, dans le milieu de culture complet selon l'invention.

Ainsi, l'expression milieu complet s'étend également à une forme de milieu complet dite concentrée au regard du fait qu'elle est obtenue à l'issue d'une évaporation partielle de l'eau constituant un milieu de fermentation dans lequel ont été réalisée consécutivement la culture du microorganisme correspondant et la lyse cellulaire de celui-ci. Bien entendu, cette évaporation est conduite dans des conditions opératoires ajustées pour ne pas altérer l'intégrité des constituants non aqueux formant ce milieu complet.

### Composition milieu de fermentation

Par définition, un milieu de fermentation ou encore de culture est un support qui permet la culture et donc selon le cas, le développement de cellules, de bactéries, de levures. En principe, les cellules trouvent dans ce milieu les composants indispensables pour leur multiplication en grand nombre rapidement, mais aussi parfois des éléments qui permettront de privilégier le développement d'un genre bactérien spécifique ou d'une famille particulière, en l'occurrence une bactérie appartenant au genre *Vitreoscilla sp.* (Notamment, espece: *Vitreoscilla filiformis).*

Sa composition doit donc satisfaire les exigences nutritives du microorganisme considéré et nécessaire à la prolifération de celui-ci.

Plus précisément, la composition de ce milieu de culture doit :
- couvrir les besoins en ions minéraux, en facteurs de croissance, apporter la source de carbone et d'énergie ;
- présenter un pH voisin du pH optimal et;
- présenter une force ionique optimale (le milieu peut être isotonique mais ce n'est pas obligatoire).

Ainsi, la composition d'un milieu de fermentation convenant à l'invention comprend au moins :
- une source de carbone et d'énergie, généralement figurée par un sucre et avantageusement le glucose,
- une source de potassium et de phosphore à l'image par exemple du K₂HPO₄,
- une source d'azote et de soufre pouvant être représentée par le composé (NH₄)₂SO₄;
- une source de magnésium telle que par exemple MgCl₂,
- une source de calcium telle que par exemple CaCl₂,
- une source de fer et plus particulièrement du citrate de fer, le citrate ayant pour rôle de maintenir le fer en solution,
- une source d'oligo-éléments choisis notamment parmi les sels de Cu, Zn, Co, Ni, B, Ti,
- une source d'eau, généralement stérile, indispensable à toute forme de vie,
- un tampon pH pouvant être figuré par le KH₂PO₄.

A défaut de présence de l'un de ces composants, les bactéries ne se développent pas, car elles ne peuvent par elles-mêmes suppléer à son absence.

A titre illustratif d'un milieu de fermentation convenant au développement d'un microorganisme conforme à l'invention, il peut notamment être fait mention du milieu figurant en exemple 1 ci-après.

Une quantité efficace du microorganisme considéré selon l'invention y est introduite et l'ensemble est placé dans des conditions propices à sa prolifération.

Pour obtenir un lysat de bactéries appartenant au genre *Vitreoscilla sp.,* dans un milieu de fermentation complet, selon un procédé comprenant une étape de culture desdites bactéries, l'homme du métier peut se référer notamment à l'exemple 1.

Avantageusement, pour obtenir un actif selon l'invention, c'est-à-dire un lysat de bactéries appartenant au genre *Vitreoscilla sp.* dans un milieu de fermentation complet, la biomasse (cellules bactériennes après la phase de croissance, présentes dans le milieu dans lequel elles ont été cultivées) est congelée, par exemple à une température de -20°C, puis est stérilisée, de préférence par la chaleur, en particulier en soumettant la biomasse préalablement congelée à une étape de chauffage à une température supérieure à 100°C. A titre illustratif, l'étape de stérilisation de la biomasse peut être réalisée par autoclavage, par exemple à une température de 121°C.

Comme il ressort de ce qui précède, à l'issue de cette culture dudit microorganisme celui est transformé à l'état de lysat directement au sein du milieu de fermentation ayant servi à sa culture.

### Lyse des bactéries pour l'obtention d'un actif selon l'invention

Un lysat désigne communément un matériau obtenu à l'issue de la destruction ou dissolution de cellules biologiques par un phénomène dit de lyse cellulaire provoquant ainsi la libération des constituants biologiques intracellulaires et cellulaires naturellement contenus dans les cellules biologiques considérées.

Au sens de la présente invention, le terme lysat désigne l'intégralité du lysat obtenu par lyse du microorganisme concerné, à savoir une bactérie appartenant au genre *Vitreoscilla sp.* (notamment espece: *Vitreoscilla filiformis*)*.*

Le lysat mis en oeuvre est donc formé de l'intégralité de ses constituants biologiques intracellulaires notamment ses métabolites et les constituants des parois et membranes cellulaires générés lors de sa lyse cellulaire.

Au sens de l'invention, le terme « métabolite » désigne toute substance issue du métabolisme du microorganisme considéré selon l'invention.

Cette lyse cellulaire peut être accomplie par différentes technologies, telles que par exemple un choc osmotique, un choc thermique, par ultrasons, ou encore sous contrainte mécanique de type centrifugation.

Plus particulièrement, ce lysat peut être obtenu selon la technologie décrite dans le brevet US 4, 464,362, et notamment selon le protocole suivant.

Il est procédé à une désintégration aux ultra-sons du milieu de fermentation ayant servi à la culture de microorganisme considéré et contenant donc ledit microorganisme afin d'y libérer les fractions cytoplasmiques et cytosoliques, les fragments de paroi cellulaire et les produits issus du métabolisme de ce microorganisme. Tous ces composants y sont ensuite préservés dans leur distribution naturelle sous une forme stabilisée dans le milieu de fermentation dit complet.

En conséquence, l'actif considéré selon l'invention peut être obtenu via un procédé consistant en :
- la culture d'au moins une bactérie appartenant au genre Vitreoscilla sp. (notamment espèce: Vitreoscilla filiformis) sur un milieu de fermentation dans des conditions propices à la prolifération de ladite bactérie, et
- la lyse cellulaire desdites bactéries dans ledit milieu de fermentation.

### Bactéries appartenant au genre Vitreoscilla sp. (notamment especes: Vitreoscilla filiformis)

Comme précisé ci-dessus, le microorganisme considéré selon l'invention à l'état de lysat est une bactérie filamenteuse non synthétique telle que définie selon la classification de Bergey's Manual of Systematic Bacteriology (Vol. 3, sections 22 et 23, 9ème édition, 1989), et appartenant au genre Vitreoscilla sp. (Notamment espece: *Vitreoscilla filiformis*)*.*

Comme il ressort des exemples présentés ci-après, les inventeurs ont découvert de manière inattendue qu'une telle bactérie mise en oeuvre sous la forme de son lysat formulé dans un milieu de fermentation complet au sens de la présente invention présente des propriétés supérieures en terme d'efficacité à celles de la biomasse obtenue à partir du même milieu de fermentation.

Plus particulièrement, il s'agit d'une bactérie appartenant au genre Beggiatoa, Vitreoscilla, *Flexithrix* ou *Leucothrix.*

Parmi les bactéries utilisables, on peut citer par exemple, *Vitreoscilla filiformis* (ATCC 15551). ,.

Selon une variante préférée de l'invention, il s'agit de la bactérie *Vitreoscilla filiformis.*

### Applications

Comme signifié précédemment, l'actif considéré selon la présente invention est particulièrement intéressant au regard de son efficacité à l'égard de certains désordres cosmétiques liés à des états pelliculaires du cuir chevelu, associées à une prévalence de microorganismes pathogènes sur le cuir chevelu et/ou un déséquilibre de l'écoflore du cuir chevelu.

Un actif conforme à l'invention est avantageusement utilisé en vue de traiter et/ou prévenir les états pelliculaires et les troubles du cuir chevelu associés.

Ainsi, un cuir chevelu présentant une sécheresse excessive ou une sécrétion excessive de sébum, peut manifester un état pelliculaire, lequel, selon le cas, peut se caractériser par la présence de pellicules sèches ou grasses, voire un prurit et/ou une inflammation de l'épiderme.

Les états pelliculaires secs traduisent une xérose du cuir chevelu, le cas échéant associée à un renouvellement excessivement rapide de son *stratum corneum.*

Les pellicules sèches sont généralement de petites tailles, blanches ou grises, et se répartissent sur le cuir chevelu et les vêtements générant un effet visuel inesthétiques.

Quant aux états pelliculaires gras, ils se manifestent sous une des formes sub-pathologiques de dermites séborrhéiques.

Lors des états pelliculaires du cuir chevelu, la barrière cutanée est déséquilibrée, son intégrité et son hydratation sont altérées et son écoflore perturbée. La peau du cuir chevelu est irritée et prurigineuse, fragile, moins hydratée et sensible aux infections.

Comme il ressort des exemples ci-après, la présente invention permet avantageusement de disposer d'un nouvel actif particulièrement efficace à l'égard des états pelliculaires.

Avantageusement, un actif conforme à l'invention est susceptible de réduire le risque de survenue d'effets secondaires .

L'actif considéré selon l'invention permet encore avantageusement de restaurer un cuir chevelu sain, en parfaite homéostasie et de rétablir une écoflore équilibrée

En conséquence, les utilisations cosmétiques, procédés cosmétiques et compositions selon l'invention, s'avèrent tout particulièrement efficaces :
- pour améliorer l'hygiène et/ou le soin du cuir chevelu, et en particulier
- pour prévenir et/ou traiter les états pelliculaires, qu'ils soient secs ou gras, du cuir chevelu,
- pour prévenir et/ou traiter les désordres, notamment esthétiques, du cuir chevelu associés à un excès d'excrétion et/ou de sécrétion de sébum,
- pour conférer une sensation de bien être au cuir chevelu,
- pour améliorer le confort du cuir chevelu,
- pour améliorer et/ou rétablir les défenses antimicrobiennes endogènes du cuir chevelu,
- pour préserver et/ou renforcer l'intégrité des fonctions barrières du cuir chevelu,
- pour rétablir une écoflore équilibrée du cuir chevelu, et/ou
- pour prévenir et/ou traiter les prurits et démangeaisons d'origine séborrhéiques associés aux états pelliculaires du cuir chevelu.

De manière générale, la présente invention vise une composition comprenant, comme unique agent actif anti-pelliculaire, un lysat de bactérie(s) appartenant au genre *Vitreoscilla sp.,* dans un milieu de fermentation complet

Toutefois, l'invention vise également une composition comprenant, outre un lysat conforme à l'invention dans un milieu de fermentation complet, au moins un actif antipelliculaire additionnel distinct et/ou au moins un actif cosmétique additionnel.

### Actif cosmétique additionnel

Un procédé selon l'invention peut comprendre, outre l'administration d'un actif selon l'invention sous une forme ou non associée à un actif antipelliculaire, l'administration d'au moins un troisième actif cosmétique.

Avantageusement, un tel actif cosmétique additionnel peut être destiné à exercer un effet cosmétique, de soin ou d'hygiène sur le cuir chevelu, voire être destiné à renforcer la barrière cutanée du cuir chevelu.

Il peut notamment s'agir d'un microorganisme probiotique, et/ou une de ses fractions, et/ou un de ses métabolites, distinct de ladite bactérie considérée dans l'actif selon l'invention et/ou de la culture complète de ce microorganisme.

### Microorganisme probiotique

Au sens de la présente invention, on entend par « microorganisme probiotique », un microorganisme vivant qui, lorsqu'il est consommé en quantité adéquate, a un effet positif sur la santé de son hôte (« Joint FAO/WHO Expert Consultation on Evaluation of Health and Nutritional Properties of Probiotic in Food Including Powder Milk with Live Lactic Acid Bacteria, 6 octobre 2001 »), et qui peut en particulier améliorer l'équilibre microbien intestinal.

Selon un mode de réalisation, un microorganisme probiotique convenant à l'invention peut être choisi parmi des *Lactobacillus sp.,* des *Bifidobacterium sp.,* des *Cocci,* des levures, des bactéries sporulées, et leurs mélanges.

Selon un mode de réalisation, un microorganisme convenant à l'invention est préférentiellement choisi parmi :
- les bactéries lactiques : qui produisent par fermentation du sucre de l'acide lactique. Suivant leur morphologie on les divise en deux groupes :
   *Lactobacillus species : Lactobacillus acidophilus, amylovorus, casei, rhamnosus, brevis, crispatus, delbrueckii (subsp bulgaricus, lactis), fermentum, helveticus, gallinarum, gasseri, johnsonii, plantarum, reuteri, salivarius, alimentarius, curvatus, casei subsp. casei, sake, et*
   *Cocci : Enterococcus (faecalis, faecium), Lactococcus lactis (subsp lactis ou cremoris), Leuconostoc mesenteroides* subsp *dextranicum, Pediococcus acidilactici, Sporolactobacillus inulinus, Streptococcus salvarius subsp. thermophilus, Streptococcus thermophilus, Staphylococccus carnosus, Staphylococcus xylosus,*
- les bifidobactéries ou *Bifidobacterium species : Bifidobacterium adolescentis, animalis, bifidum, breve, lactis, longum, infantis, pseudocatenulatum,*
- les levures : *Saccharomyces (cerevisiae* ou encore *boulardii),*
- les autres bactéries sporulées : *Bacillus (cereus var toyo ou subtilis), Bacillus coagulans, Bacillus licheniformis, Escherichia coli strain nissle, Propionibacterium freudenreichii,*
- et leurs mélanges.

Un microorganisme convenant à l'invention peut être choisi notamment parmi les ascomycètes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* et *Lactobacillus,* et leurs mélanges.

Comme autre exemple de microorganismes probiotiques convenant à l'invention, on peut citer *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus* NCFB 1748 *; Lactobacillus amylovorus, Lactobacillus casei (Shirota), Lactobacillus rhamnosus* souche GG, *Lactobacillus brevis, Lactobacillus crispatus, bulgaricus, Lactobacillus delbrueckii subsp., lactis, Lactobacillus fermentum, Lactobacillus helveticus, Lactobacillus gallinarum, Lactobacillus gasseri, Lactobacillus johnsonii* CNCM I-1225, *Lactobacillus plantarum, Lactobacillus reuteri, Lactobacillus salivarius, Lactobacillus alimentarius, Lactobacillus curvatus, Lactobacillus casei subsp. casei, Lactobacillus sake, Lactococcus lactis, Enterococcus faecalis, Enterococcus faecium, Lactococcus lactis subsp lactis, Lactococcus lactis subsp cremoris, Leuconostoc mesenteroides* subsp *dextranicum, Pediococcus acidilactici, Sporolactobacillus inulinus, Streptococcus salvarius subsp. thermophilus, Streptococcus thermophilus, Staphylococccus carnosus, Staphylococcus xylosus, Saccharomyces cerevisiae, Saccharomyces boulardii, Bacillus cereus var toyo, Bacillus cereus var subtilis, Bacillus coagulans, Bacillus licheniformis, Escherichia coli strain nissle, Propionibacterium freudenreichii,* et leurs mélanges.

Plus particulièrement, il peut s'agir d'un microorganisme probiotique choisi parmi *Lactobacillus sp., Sporolactobacillus sp., Enterococcus sp., Lactococcus sp., Bacillus sp., Streptococcus sp., Pediococcus sp., Leuconostoc sp.* ou *Bififobacterium sp.,* et en particulier choisi parmi *Lactobacillus sp.* et *Bifidobacterium sp.,* et leurs mélanges.

A titre illustratif de ces microorganismes probiotiques, on peut plus particulièrement citer les *Lactobacillus johnsonii, Lactobacillus reuteri, Lactobacillus rhamnosus, Lactobacillus paracasei, Lactobacillus casei, Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum,* et leurs mélanges.

Les espèces convenant tout particulièrement sont *Lactobacillus johnsonii, Lactobacillus paracasei, Bifidobacterium adolescentis, Bifidobacterium longum* respectivement déposés suivant le traité de Budapest avec l'Institut Pasteur (28 rue du Docteur Roux, F-75024 Paris cedex 15) les 30/06/92, 12/01/99, 15/04/99 et le 15/04/99 sous les désignations suivantes CNCM I-1225, CNCM I-2116, CNCM I-2168 et CNCM I-2170, et le genre *Bifidobacterium lactis (Bb 12)* (ATCC27536) ou *Bifidobacterium longum* (BB536). La souche de *Bifidobacterium lactis* (ATCC27536) peut être obtenue chez Hansen (Chr. Hansen A/S, 10-12 Boege Alle, P.O. Box 407, DK-2970 Hoersholm, Danemark).

Avantageusement, un microorganisme convenant à l'invention, à titre d'actif annexe, peut être un microorganisme probiotique à acide lactique.

Selon un mode de réalisation préféré, un microorganisme probiotique convenant à l'invention peut en particulier être un microorganisme du genre *Lactobacillus sp.*

De manière préférée, un microorganisme du genre *Lactobacillus sp.* convenant à l'invention peut être choisi parmi les espèces *Lactobacillus johnsonii, Lactobacillus reuteri, Lactobacillus paracasei, Lactobacillus casei,* et leurs mélanges.

Selon un mode de réalisation préféré, un microorganisme convenant à l'invention peut être un *Lactobacillus paracasei.*

Un microorganisme convenant à l'invention peut en particulier être la souche *Lactobacillus paracasei* ST11 déposée suivant le traité de Budapest auprès de l'Institut Pasteur (28 rue du Docteur Roux, F-75024 Paris cedex 15) le 12/01/99 sous la désignation CNCM I-2116, et/ou une de ses fractions et/ou un de ses métabolites.

Selon un autre mode de réalisation préféré, un microorganisme probiotique convenant à l'invention peut en particulier être un microorganisme du genre *Bifidbacterium sp.,* et en particulier *Bifidobacterium longum,* notamment *Bifidobacterium longum* (BB536).

Un tel microorganisme peut être formulé dans une composition à raison d'au moins 0,0001 % exprimé en poids sec, en particulier à raison de 0,0001 à 20 % et plus particulièrement à raison de 0,001 à 15 % en poids, en particulier de 0,01 à 10 % en poids, et notamment de 0,1 % à 2 % en poids par rapport au poids total de la composition le contenant.

### Formulation galénique

Une composition contenant l'actif selon l'invention peut être administrée par voie orale ou topique.

Une composition selon l'invention comprend avantageusement une quantité de lysat de bactérie(s) appartenant au genre *Vitreoscilla sp.,* dans un milieu de fermentation complet allant de 0,001% à 10% en poids, par rapport au poids total d'extrait sec de ladite composition. Dans certains modes de réalisation, une composition selon l'invention comprend avantageusement une quantité de lysat de bactérie(s) appartenant au genre *Vitreoscilla sp.* dans un milieu de fermentation complet allant de 0,01% à 5% en poids, par rapport au poids total d'extrait sec de ladite composition. Dans d'autres modes de réalisation, une composition selon l'invention comprend avantageusement une quantité de lysat de bactérie(s) appartenant au genre *Vitreoscilla sp.,* dans un milieu de fermentation complet allant de 0, 1% à 1% en poids, par rapport au poids total d'extrait sec de ladite composition.

Elle peut donc se présenter sous toutes les formes galéniques normalement disponibles pour le mode d'administration retenu.

Le support peut être de nature diverse selon le type de composition considérée.

En ce qui concerne plus particulièrement les compositions destinées à une administration par voie topique externe, il peut s'agir de solutions aqueuses, hydroalcooliques ou huileuses, de solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions, de consistance molle, semi-solide ou solide, du type crème, de gel aqueux ou anhydre, de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de protection, de traitement ou de soin des lotions, gels ou mousses pour le soin du cuir chevelu, comme des lotions de nettoyage ou de désinfection du cuir chevelu.

Elles peuvent être utilisées pour le cuir chevelu sous forme de solutions, de crèmes, de gels, d'émulsions, de mousses ou encore sous forme de compositions pour aérosol contenant également un agent propulseur sous pression.

Une composition par voie topique selon l'invention peut avantageusement être formulée sous toute forme galénique convenant au soin capillaire, notamment sous forme d'une lotion capillaire, d'un shampoing, notamment antipelliculaire, d'un après-shampoing, d'un démêlant, d'une crème ou d'un gel capillaire, d'une laque coiffante, d'une lotion de mise en plis, d'une lotion traitante, d'une composition de teinture (notamment d'oxydation) éventuellement sous forme de shampooing colorant, d'une lotion restructurante pour cheveux, d'une composition de permanente, d'une lotion ou d'un gel antichute, d'un shampoing antiparasitaire, ou d'un shampoing traitant, notamment anti-séborrhéique, d'un produit de soin du scalp notamment anti-irritant, anti-âge, restructurant, ou activateur de la circulation sanguine.

Lorsqu'une composition de l'invention est une émulsion, la proportion de la phase grasse peut aller de 5 à 80 % en poids, et de préférence de 10 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, l'huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras comme par exemple l'acide stéarique et comme par exemple des cires notamment de paraffine, de carnauba et la cire d'abeilles. On peut aussi utiliser des composés siliconés comme les huiles siliconées et par exemple les cyclométhicone et diméthicone, les cires, les résines et les gommes siliconées.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO® par la société HENKEL, le mélange de PEG-6/PEG-32/Glycol Stéarate vendu sous la dénomination de Tefose® 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

Comme solvants utilisables dans l'invention, on peut citer les alcools inférieurs, notamment l'éthanol et l'isopropanol, le propylène glycol.

La composition de l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305® par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

### Procédé de traitement cosmétique

Comme indiqué précédemment, un procédé selon l'invention peut être mis en oeuvre, par voie topique, notamment par application sur le cuir chevelu d'au moins un lysat de bactérie appartenant au genre Vitreoscilla sp. (notamment, espèces: *Vitreoscilla filiformis*) dans un milieu de fermentation complet à titre d'actif pour prévenir et/ou traiter les états pelliculaires du cuir chevelu, notamment les états pelliculaires associés à une prolifération de microorganismes pathogènes sur le cuir chevelu et/ou un déséquilibre de l'écoflore du cuir chevelu, et plus particulièrement d'une composition cosmétique telle que définie précédemment.

Avantageusement, un procédé de l'invention par voie topique peut comprendre l'application d'une composition conforme à l'invention, par exemple sous la forme de crèmes de nettoyage, des lotions, ou des gels de traitement ou de soin pour le cuir chevelu, des gels ou mousses pour le soin du cuir chevelu, comme les lotions de nettoyage ou de désinfection ou des shampoings. Selon une variante de réalisation, le procédé de l'invention peut comprendre l'application topique sur le cuir chevelu d'au moins un actif selon l'invention par exemple sous forme de shampoing, de gels, de sérums, de lotions.

Un procédé cosmétique topique selon l'invention peut être mis en oeuvre de façon journalière par exemple, à raison par exemple d'une administration unique par jour ou d'une administration deux fois par jour, par exemple une fois le matin et une fois le soir.

Un procédé cosmétique topique selon l'invention peut être mis en oeuvre sur une période de temps variant d'une semaine à plusieurs semaines, voire plusieurs mois, cette période pouvant par ailleurs être répétée après des périodes de non traitement, pendant plusieurs mois voire plusieurs années.

A titre d'exemple, l'administration par voie topique d'un composé selon l'invention peut être répétée, par exemple, 2 à 3 fois par semaine, ou plus, et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre ... et ... » incluent les bornes inférieure et supérieure précisées.

Les exemples ci-après sont présentés à titre illustratif et non limitatif du domaine de l'invention.

### EXEMPLES

### EXEMPLE 1

### Préparation d'un actif conforme à l'invention

La réalisation du milieu complet de fermentation est obtenu au moyen d'une culture de la souche *Vitreoscilla filiformis,* dans son milieu de culture complet.

Le milieu de culture initial pour l'obtention du milieu de fermentation complet est de composition décrite dans le Tableau 1 ci-dessous.

**Tableau 1**

| Nom chimique | [c] |
|---|---|
| Extrait autolytique de levure | 4 g/l |
| Peptone papainique de soja F | 3 g/l |
| Glucose - Roférose | 3 g/l |
| KH₂PO₄ | 0.088 g/l |
| CaCl₂ | 0.050 g/l |
| CuSO₄, 5 H₂O | 60 µ g/l |
| MnSO₄, 1H₂O | 152 µ g/l |
| KI | 20 µg/l |
| ZnSO₄, 7H₂O | 200 µg/l |
| AlCl₃, 6H₂O | 100 µ g/l |
| eau osmosée | qsp 1 l |

Pour obtenir un actif cosmétique selon l'invention, c'est-à-dire dans cet exemple un lysat de bactéries *Vitreoscilla filiformis* dans un milieu de fermentation complet, on a procédé comme décrit ci-après.

La souche *Vitreoscilla filiformis* a été obtenue auprès de l'ATCC (souche 15551). Cette souche est cultivée dans un milieu de culture particulier 2BHG2, dont la composition est donnée ci-dessus.

La biomasse est obtenue par une culture continue dans un bioréacteur de 3000 litres efficaces. Un taux de croissance d'environ 70% du µmax (µ = 0.12 H-1) est enregistré durant la phase de production en continu. Durant cette étape, nous contrôlons le pH (7.00), la température (26°C) et l'oxygène dissous (0/5%). L'extraction et la séparation des cellules est obtenu par centrifugation (10 000 g/ 20mn). La biomasse est congelée à - 20°C puis la biomasse est conditionnée en poches (rupture de stérilité) et est stabilisée par stérilisation à 121°C pendant 30mn. La biomasse est ensuite appelée Vfe.

Les analyses de spécification de la biomasse sont les suivantes :
- Résidu fixe à 105°C (g/100g) : 4.0 à 4.5%
- Teneur en azote total sur MA : 10.0 à 14.0%
- Microbiologie : 0 germe/g
- Teneur en acide 3-hydroxybutyrique : 2 à 10 g/l (<10 g/l)
- pH de la solution en état : 4 à 5.

Le milieu de fermentation est la culture complète, obtenue durant la fermentation en continu. Le glucose du milieu de départ a été consommé par les µ-organismes (µ-contrôlé par la source de carbone), ainsi que divers éléments des peptones et extrait de levure apportés au départ. Le milieu de fermentation actuellement testé est prélevé directement du fermenteur, puis subit le barème de stérilisation.

Le MF qui est la culture complète non concentrée (0.7 à 0.9% de MS) est autoclavée (30 mn 121°C), ainsi que le lysat (4.0 à 4.5% de MS).

Dans les exemples qui suivent, l'actif selon l'invention (le lysat de bactéries *Vitreoscilla filiformis* dans un milieu de fermentation complet, aussi désigné « MF ») ainsi qu'un actif comparatif constitué du lysat bactérien isolé en l'absence du milieu de fermentation complet, ont fait l'objet de tests cliniques sur têtes afin de vérifier leurs activités antipelliculaires, par comparaison avec une formulation de shampoing anti-pelliculaire de référence. On a testé notamment deux formulations de shampoing antipelliculaires de référence, respectivement une première formulation de shampoing de référencecomprenant de l'octopyrox et une seconde formulation de shampoing de référence comprenant du ZnPT.

### EXEMPLE 2

Dans l'exemple 2, on a comparé les effets sur les états pelliculaires du cuir chevelu (i) d'une formulation de shampoing comprenant un actif anti-pelliculaire selon l'invention (lysat de bactéries *Vitreoscilla filiformis* dans un milieu de fermentation complet, préparé conformément à l'exemple 1) et (ii) une formulation de shampoing anti-pelliculaire de référence, exempte de l'actif anti-pelliculaire selon l'invention, comprenant un actif anti-pelliculaire conventionnel (le ZnPT).

Le lysat de bactéries dans un milieu de fermentation complet (actif anti-pelliculaire selon l'invention) tel qu'obtenu à l'exemple 1 est donc mis en oeuvre à titre d'actif antipelliculaire dans une formulation de shampoing dont la composition de base est décrite dans le Tableau 2 ci-dessous.

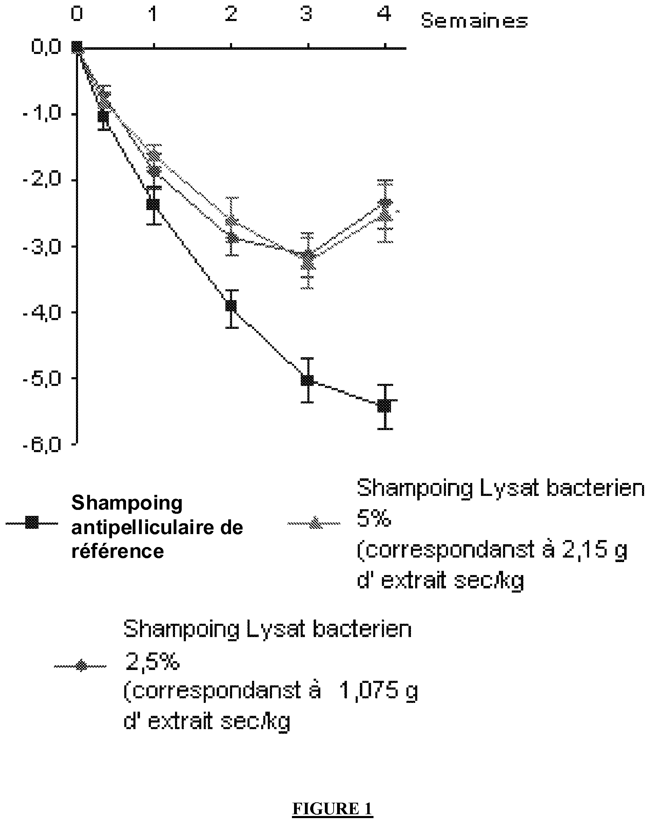

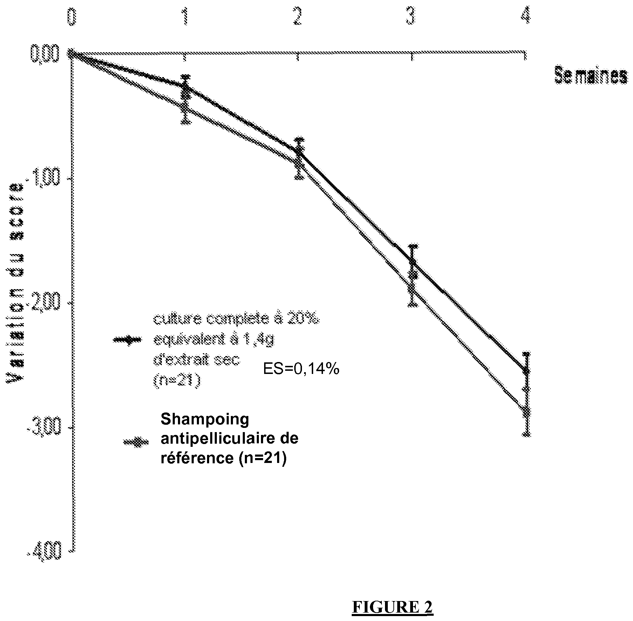

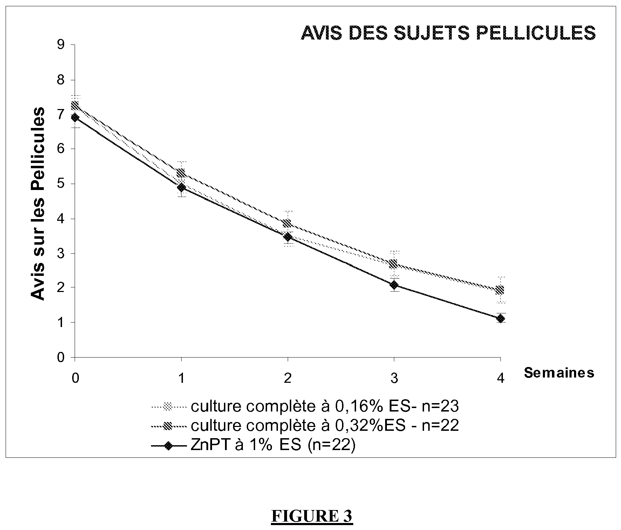

*Plusieurs études cliniques ont été réalisées,*
- *une étude clinique 1, correspondant à la* *Figure 1**,*
- *une étude clinique 2, correspondant à la* *Figure 2**,*
- *une étude clinique 3, correspondant aux* *Figures 3 à 11**.*

**Tableau 2**

| **Ingrédients** | | | **Concentration (% en poids par rapport au poids total) [A valider, SVP]** |
|---|---|---|---|
| **Nom chimique** | **Nom INCI (US)** | **Nom INCI (UE)** | |
| Lysat de *Vitreoscilla filimormis* dans leur milieu complet (culture complète à 160g/l) | | | 2 |
| Lauryl ether sulfate de sodium (2.2 OE) en solution aqueuse | Sodium laureth sulfate | Sodium laureth sulfate | 20,1 |
| Cocoyl amidopropyl betaïne en solution aqueuse | Cocamidopropyl betaïne | Cocamidopropyl betaïne | 6,3 |
| Eau | | | QSP |

La formulation de shampoing, utilisée dans la clinique 3, du Tableau 2 comprend 2% en poids de l'actif anti-pelliculaire selon l'invention (lysat de bactéries dans un milieu de fermentation complet, à une concentration de 160g/l) par rapport au poids total de la composition, c'est-à-dire 0,32% en poids dudit actif anti-pelliculaire, par rapport au poids total d'extrait sec de ladite composition.

De façon équivalente, la formulation de shampoing utilisée dans la clinique 3 comprend 1% en poids de l'actif anti-pelliculaire selon l'invention (lysat de bactéries dans un milieu de fermentation complet, à une concentration de 160g/l) par rapport au poids total de la composition, c'est-à-dire 0,16% en poids dudit actif anti-pelliculaire, par rapport au poids total d'extrait sec de ladite composition.

De façon équivalente, la formulation de shampoing utilisée dans la clinique 1 comprend 2.5% et 5% en poids de l'actif lysat bactérien (lysat de bactéries exempt du milieu de fermentation complet) par rapport au poids total de la composition, c'est-à-dire respectivement 0.1075% et 0.215% en poids dudit actif, par rapport au poids total d'extrait sec de ladite composition.

De façon équivalente, la formulation de shampoing utilisée dans la clinique 2 comprend 20% en poids de l'actif anti-pelliculaire selon l'invention (lysat de bactéries dans un milieu de fermentation complet, à une concentration de 7.5g/l) par rapport au poids total de la composition, c'est-à-dire 0.14%% en poids dudit actif anti-pelliculaire, par rapport au poids total d'extrait sec de ladite composition.

On précise que la quantité en % en poids du lysat dans son milieu de fermentation complet varie de 0.1% à 0.5% en extrait sec

A titre comparatif, on a testé la formulation de shampoing de référence décrite dans le Tableau 3 ci-dessous, qui comprend l'actif anti-pelliculaire zinc pyrythione (ZnPT) présent à 2% en poids, par rapport au poids total de la composition, c'est-à-dire 1% d'extrait sec en poids zinc pyrythione, par rapport au poids total d'extrait sec de ladite composition.

**Tableau 3**

| **Ingrédients** | | | **Concentration (% en poids par rapport au poids total)** |
|---|---|---|---|
| **Nom chimique** | **Nom INCI (US)** | **Nom INCI (UE)** | |
| Pyrythione de zinc en dispersion aqueuse | Zinc pyrythione | Zinc pyrythione | 2,083, ce qui correspond à un ES en poids de 1% |
| Lauryl ether sulfate de sodium (2.2 OE) en solution aqueuse | Sodium laureth sulfate | Sodium laureth sulfate | 20,1 |
| Cocoyl amidopropyl betaïne en solution aqueuse | Cocamidopropyl betaïne | Cocamidopropyl betaïne | 6,3 |
| Eau | | | QSP |

### Ptotocole

Le traitement consiste à appliquer par voie topique la formule testée pendant quatre semaines.

### Etude clinique 1 :

Cette étude a été engagée pour comparer (i) un shampoing comprenant un lysat bactérien isolé, exempt de milieu de fermentation complet, utilisé à 2,5% en poids (correspondant à 0.1075% d'extrait sec), (ii) un shampoing comprenant un lysat bactérien isolé, exempt de milieu de fermentation complet, utilisé à 5% en poids (correspondant à 0.215% d'extrait sec), et (iii) la formule de shampoing anti-pelliculaire de référence.

Cette étude a été réalisée sur 60 sujets adultes de sexe masculin dont l'âge est compris entre 18 et 60 ans, et qui ont été identifiés à la suite d'une évaluation clinique de leur état pelliculaire, classé de modéré à sévère, avec des scores supérieurs ou égaux à 5 (sur une échelle de 0 à 9) en présence de squames adhérentes sur au moins deux quarts de la tête, et de leur érythème du cuir chevelu.

Les 60 sujets ont été répartis en 3 groupes parallèles de 20 sujets, avec chacun des groupes étant traité avec une seule des deux compositions + un groupe temoin

### Etude clinique 2 :

Cette étude a été engagée pour comparer de (i) un shampoing comprenant l'actif de l'invention à 20% en poids (correspondant à 0.14% d'extrait sec), et (ii) la formule de shampoing de référence. L'actif de l'invention st sous une forme à 7.5g/l.

Cette étude a été réalisée sur 42 sujets adultes de sexe masculin dont l'âge est compris entre 18 et 60 ans, et qui ont été identifiés à la suite d'une évaluation clinique de leur état pelliculaire, classé de modéré à sévère, avec des scores supérieurs ou égaux à 5 (sur une échelle de 0 à 9) en présence de squames adhérentes sur au moins deux quarts de la tête, et de leur érythème du cuir chevelu.

Les 42 sujets ont été répartis en 2 groupes parallèles de 21 sujets, avec un groupe étant traité avec une composition présentant l'actif de l'invention+ un groupe témoin avec l'actif antipelliculaire de référence.

### Etude clinique 3 :

Cette étude a été engagée pour comparer de (i) un shampoing comprenant l'actif de l'invention à 0.16% en extrait sec, (ii) un shampoing comprenant l'actif de l'invention à 0.32% en extrait sec et (iii) la formule de shampoing de référence, comprenant du zinc pyrythione à 1% en extrait sec. L'actif de l'invention est sous une forme à 160g/l.

Cette étude a été réalisée sur 67 sujets adultes de sexe masculin dont l'âge est compris entre 18 et 60 ans, et qui ont été identifiés à la suite d'une évaluation clinique de leur état pelliculaire, classé de modéré à sévère, avec des scores supérieurs ou égaux à 5 (sur une échelle de 0 à 9) en présence de squames adhérentes sur au moins deux quarts de la tête, et de leur érythème du cuir chevelu.

Les 67 sujets ont été répartis en 3 groupes parallèles de 22 sujets, avec chacun des groupes étant traité avec une seule des deux compositions + un groupe témoin

### Evaluation clinique de l'état pelliculaire du cuir chevelu (scores)

Les effets sur les pellicules ont été testés et appréciés par comparaison des groupes à J2, J7, J15, J21 et J28 au travers d'évaluations cliniques réalisées sur les paramètres suivants : pellicules libres, squames adhérents et érythème, et le score global a été calculé.

Ces évaluations ont été effectuées par des dermatologues qualifiés selon les techniques habituellement mises en oeuvre dans le domaine.

A chaque visite, l'état pelliculaire, pellicules libres et squames adhérentes, a été scoré, pour chaque item, par l'investigateur en utilisant une échelle de 0 - 9, un score de 0 étant attribué en l'absence d'un état pelliculaire et un score de 9 étant attribué pour un état pelliculaire très sévère.

Les résultats sont exprimés par la différence entre (i) la valeur du score avant le début du traitement et (ii) la valeur du score au moment de chaque mesure.

Auto-évaluation de l'état pelliculaire du cuir chevelu par les sujets.

Les effets sur les pellicules ont été testés et appréciés par comparaison des groupes à J2, J7, J15, J21 et J28 au travers d'auto-évaluations cliniques réalisées sur les sujets eux-mêmes. Ces auto-évaluations représentent « l'Avis des sujets ».

Les résultats sont exprimés par la valeur du score pelliculaire attribué par le sujet lui-même.

### Evaluation clinique des démangeaisons du cuir chevelu

Les effets sur les démangeaisons du cuir chevelu ont été testés et appréciés par comparaison des groupes à J2, J7, J15, J21 et J28 au travers d'évaluations cliniques réalisées selon un protocole conventionnel.

Ces évaluations ont été effectuées par des dermatologues qualifiés selon les techniques habituellement mises en oeuvre dans le domaine.

A chaque visite, les démangeaisons du cuir chevelu ont été scorées par l'investigateur en utilisant une échelle de 0 - 9, un score de 0 étant attribué en l'absence de démangeaison et un score de 9 étant attribué en présence de démangeaisons très sévères.

Les résultats sont exprimés par la différence entre (i) la valeur du score avant le début du traitement et (ii) la valeur du score au moment de chaque mesure.

### Résultats

Les résultats obtenus sont illustrés en figures 1 à 4.

La figure 1 illustre les résultats de la comparaison des effets sur les pellicules de (i) un shampoing comprenant un lysat bactérien isolé, exempt de milieu de fermentation complet, utilisé à 2,5% en poids (correspondant à 0.1075% d'extrait sec), (ii) un shampoing comprenant un lysat bactérien isolé, exempt de milieu de fermentation complet, utilisé à 5% en poids (correspondant à 0.215% d'extrait sec), et (iii) la formule de shampoing anti-pelliculaire de référence.

De la comparaison des résultats de la figure 1, il ressort que les deux formulations de shampoing comprenant un lysat bactérien isolé, utilisé à 0.1075% et 0.215% en extrait sec, procurent chacun un score global significativement inférieur au score global obtenu avec la formulation de shampoing de référence. en conséquence, les résultats de la figure 1 montrent que les formulations de shampoing comprenant un lysat bactérien isolé, exempt du milieu de fermentation, exercent un effet anti-pelliculaire très faible, et même pratiquement nul, en comparaison avec la formulation de shampoing de référence.

La figure 2 illustre les résultats de la comparaison des effets sur les pellicules de (i) un shampoing comprenant l'actif de l'invention à 20% en poids (correspondant à 0.14% d'extrait sec), et (ii) la formule de shampoing de référence.

Les résultats de la figure 2 montrent que la formulation de shampoing conforme à l'invention présente une activité anti-pelliculaire équivalente à celle de la formule de référence et ceci à un équivalent en extrait sec de 0.14%, c'est-à-dire à une concentration d'actif pour laquelle un effet faible ou aucun effet n'est observé avec les formulations de shampoing comparatives comprenant un lysat bactérien isolé, exempt de milieu de fermentation complet (voir les résultats de la figure 1).

La figure 3 illustre les résultats de la comparaison des effets sur les pellicules de (i) un shampoing comprenant l'actif de l'invention à 0.16% en extrait sec, (ii) un shampoing comprenant l'actif de l'invention à 0.32% en extrait sec et (iii) la formule de shampoing de référence, comprenant du zinc pyrythione à 1% en extrait sec.

Les résultats de la figure 3 montrent qu'une formulation de shampoing comprenant 0.16% en extrait sec d'actif selon l'invention produit un effet anti-pelliculaire équivalent à la formulation de shampoing de référence qui comprend 1% en poids d'actif conventionnel (voir aussi étude statistique de l'EXEMPLE 6).

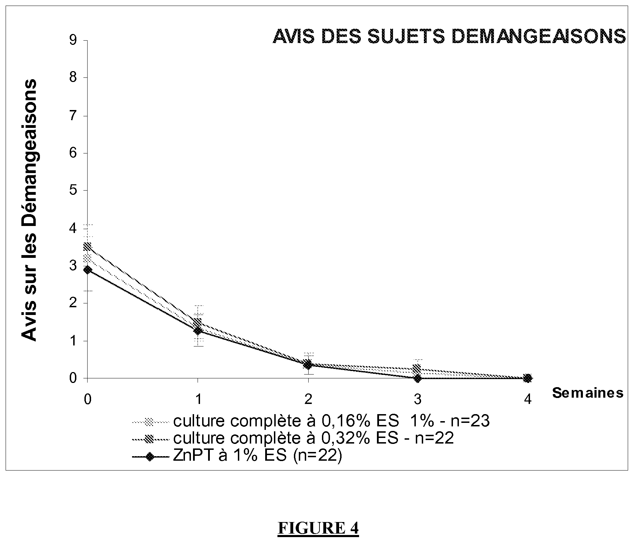

La figure 4 illustre les résultats de la comparaison des effets sur les démangeaisons du cuir chevelu de (i) un shampoing comprenant l'actif de l'invention à 0.16% en extrait sec, (ii) un shampoing comprenant l'actif de l'invention à 0.32% en extrait sec et (iii) la formule de shampoing de référence, comprenant du zinc pyrythione à 1% en poids

Les résultats de la figure 4 montrent qu'une formulation de shampoing comprenant 0.16% et 0.32% en extrait sec d'actif selon l'invention produit un effet contre les démangeaisons du cuir chevelu équivalent à la formulation de shampoing de référence qui comprend 1% en poids d'actif conventionnel.

### EXEMPLE 3

### Donnée de CMI négative pour montrer que l'effet n'est pas antifongique

Mettre en contact le produit à tester et la suspension de Malassezia. Déposer le mélange à la surface du milieu gélosé. Etaler et récupérer l'excédent avant incubation. Incuber au moins 5 jours à 30°C.

Les produits sont mis en solution dans du Leeming et Notman modifié (LNm) liquide et les essais sont réalisés en duplicate. La culture complète étant à 7,5g/l ou 150g/l (version concentrée) en matière sèche.

La culture complète a été testée à 10% (pour la concentration à 7.5g/l) et à 1% (pour la concentration à 150g/l).

La référence positive est le zinc de pyrithione 1%.

Les solutions des produits sont deux fois plus concentrées pour tenir compte de la dilution au ½ lors de la mise en contact avec la suspension de Malassezia.

**Tableau 8**

| **Souche** | **DO 550nm** | **Concentrations cfu/ml** |
|---|---|---|
| Malassezia globosa CBS 7708 | 1,369 | 7500 |
| Malassezia restricta CBS 7708 | 1,137 | 600 |

Les souches Malassezia globosa et restricta ont été réceptionnées sur pentes gélosées et ont été repiquées et incubées à 30° jusqu'aux essais.

Les suspensions sont réalisées dans 15 ml d'une solution tamponnée (réf AEB611294, AES).

La densité des suspensions est prise à 550 nm.

L'effet antifongique du produit référence est évalué par l'absence de croissance de la souche Malassezia testée. Cette inhibition est évaluée par rapport au témoin de croissance.

Les inhibitions sont notées de 3 à 0 par appréciation de la densité de la culture à la surface de la gélose en comparaison avec le témoin de croissance de la souche

**Tableau 9**

| **NOTATION** | **INTERPRÉTATION** |
|---|---|
| 3 | Pas de croissance |
| 2 | Croissance < à la boîte témoin |
| 1 | Croissance < à la boîte témoin |
| 0 | Croissance comparable à la boîte témoin |

**Tableau 10**

| | **Malassezia restricta** | | **Malassezia globosa** | |
|---|---|---|---|---|
| Témoin de croissance | Culture étendue densité convenable | | Culture étendue densité convenable | |
| Témoin zinc de pyrithione | 3 Inhibition totale | | 3 Inhibition totale | |
| ESSAIS (Duplicate) | B1 | B2 | B1 | B2 |
| Témoin TRIS 0,1M 50% | 0 | 0 | 0 | 0 |
| Culture complète à 10% (concentration 7.5g/l) Soit un ES = 0.075% | 0 | 0 | 0 | 0 |
| Culture complète à 1% (concentration 150g/l) Soit un ES = 0.15% | 0 | 0 | 0 | 0 |

Les produits ne présentent aucune inhibition antifongique sur Malassezia restricta et Malassezia globosa.

### EXEMPLE 4

### Effet sur l'état pelliculaire : avis sur le score global des pellicules et sur l'avis des sujets sur les pellicules

Dans cet exemple, on a évalué l'effet anti-pelliculaire d'une formulation de shampoing conforme à l'invention, par comparaison avec la formulation de shampoing de référence (comprenant l'actif zinc pyrythione).
Le lysat de bactéries Vitreoscilla *filiformis* dans le milieu de fermentation complet a été utilisé sous une forme évapoconcentrée, à des concentrations en extrait sec du lysat de 0.16% et 0.32% ES.

### Evaluation clinique de l'état pelliculaire du cuir chevelu (scores)

Les effets sur les pellicules ont été testés et appréciés par comparaison des groupes à J0, J7, J15, J21 et J28 au travers d'évaluations cliniques réalisées sur les paramètres suivants : pellicules libres, squames adhérents et érythème, et le score global a été calculé.

Ces évaluations ont été effectuées par des dermatologues qualifiés selon les techniques habituellement mises en oeuvre dans le domaine.
A chaque visite, l'état pelliculaire, pellicules libres et squames adhérentes, a été scoré, pour chaque item, par l'investigateur en utilisant une échelle de 0 - 9, un score de 0 étant attribué en l'absence d'un état pelliculaire et un score de 9 étant attribué pour un état pelliculaire très sévère.

Des auto-évaluations ont également été demandées aux sujets des différents groupes. Ces auto-évaluations sont reportées sous l'appellation « Avis des sujets ».

Les résultats sont présentés dans les figures 5 à 9.

Dans les figures 5, 6, 8 et 9, les résultats sont exprimés en valeurs absolues du score à chaque mesure.

Dans la figure 7, les résultats sont exprimés par la différence entre (i) la valeur du score avant le début du traitement et (ii) la valeur du score au moment de chaque mesure.

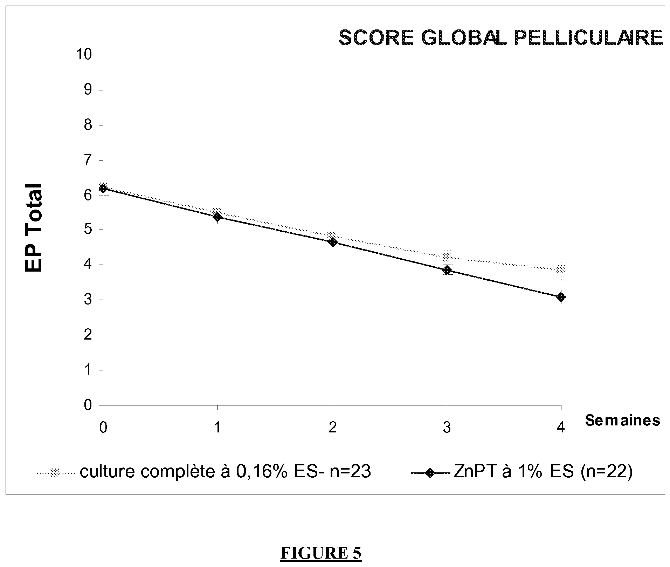

La figure 5 illustre les résultats comparatifs de score global obtenus en utilisant une formulation de shampoing conforme à l'invention comprenant 0,16% en poids de l'actif (lysat bactérien dans le milieu de fermentation complet), par rapport au poids total de l'extrait sec de la formulation. La formulation comparative de référence comprend 1% en poids d'actif ZnPT, par rapport au poids total de l'extrait sec.

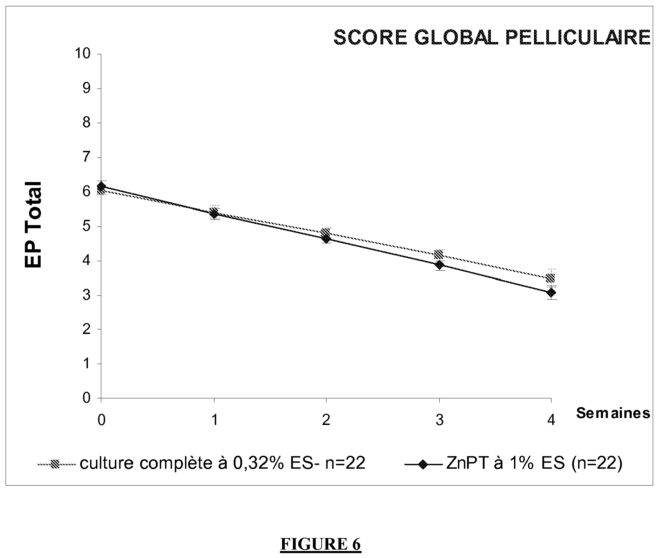

La figure 6 illustre les résultats comparatifs de score global obtenus en utilisant une formulation de shampoing conforme à l'invention comprenant 0,32% en poids de l'actif (lysat bactérien dans le milieu de fermentation complet), par rapport au poids total de l'extrait sec de la formulation. La formulation comparative de référence comprend 1% en poids d'actif ZnPT, par rapport au poids total de l'extrait sec.

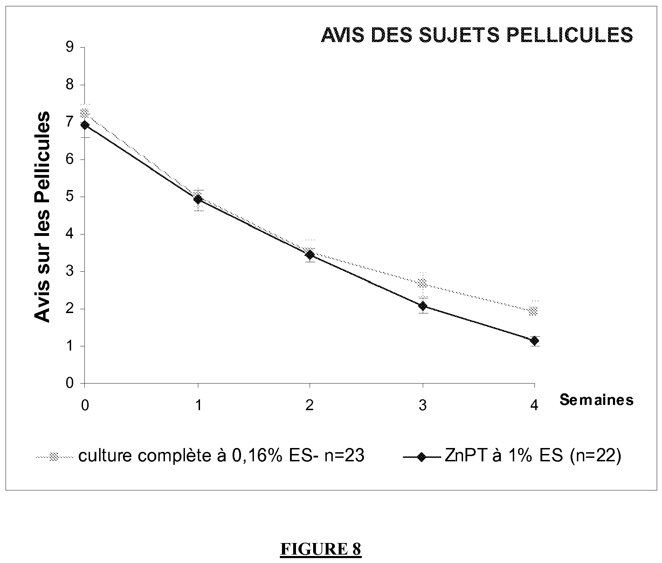

La figure 8 illustre les résultats comparatifs des avis des patients traités obtenus en utilisant une formulation de shampoing conforme à l'invention comprenant 0,16% en poids de l'actif (lysat bactérien dans le milieu de fermentation complet), par rapport au poids total de l'extrait sec de la formulation. La formulation comparative de référence comprend 1% en poids d'actif ZnPT, par rapport au poids total de l'extrait sec.

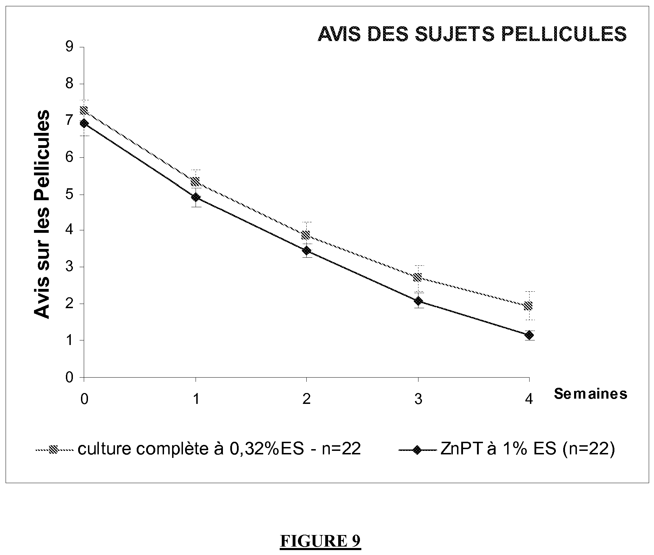

La figure 9 illustre les résultats comparatifs des avis des patients traités obtenus en utilisant une formulation de shampoing conforme à l'invention comprenant 0,32% en poids de l'actif (lysat bactérien dans le milieu de fermentation complet), par rapport au poids total de l'extrait sec de la formulation. La formulation comparative de référence comprend 1% en poids d'actif ZnPT, par rapport au poids total de l'extrait sec.

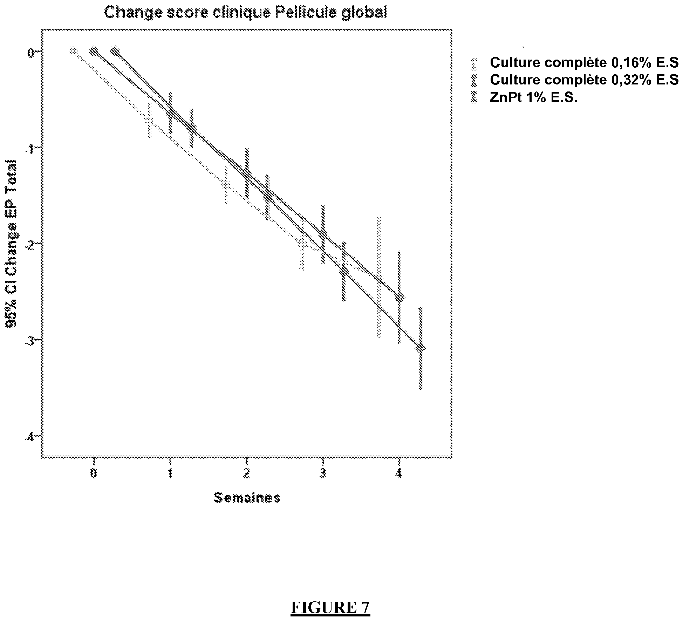

La figure 7 illustre les résultats comparatifs des différences de score global obtenus en utilisant (i) utilisant une formulation de shampoing conforme à l'invention comprenant 0,16% en poids de l'actif (lysat bactérien dans le milieu de fermentation complet), par rapport au poids total de l'extrait sec de la formulation et (ii) utilisant une formulation de shampoing conforme à l'invention comprenant 0,32% en poids de l'actif (lysat bactérien dans le milieu de fermentation complet), par rapport au poids total de l'extrait sec de la formulation. La formulation comparative de référence comprend 1% en poids d'actif, par rapport au poids total de l'extrait sec.

L'ensemble des résultats de l'exemple 6 montrent qu'une formulation de shampoing conforme à l'invention, qui comprend 0,16% d'actif anti-pelliculaire, possède une activité au moins identique, et même légèrement supérieure, à la formulation de shampoing de référence qui comprend 1% d'un actif anti-pelliculaire conventionnel.

L'ensemble des résultats de l'exemple 6 montrent qu'une formulation de shampoing conforme à l'invention, qui comprend 0,32% d'actif anti-pelliculaire, possède une activité au moins identique, et même légèrement supérieure, à la formulation de shampoing de référence qui comprend 1% d'un actif anti-pelliculaire conventionnel.

### Analyse statistique :

La culture complète à 0.32%ES n'est jamais significativement différente du ZnPt à 1 %ES.

Une fois ajusté pour prendre en compte la multiplicité des tests (3 comparaisons), la culture complète à 0.16% ES n'est jamais significativement différente du ZnPt à 1 %ES,

Les deux formulations de culture complète à 0.32% ES et 0.16% ES ne sont pas significativement différentes.

### EXEMPLE 5

### Effet sur l'état pelliculaire : avis des sujets sur les démangeaisons

Dans cet exemple, on a évalué l'effet anti-pelliculaire d'une formulation de shampoing conforme à l'invention, par comparaison avec la formulation de shampoing de référence (comprenant l'actif zinc pyrythione).

Le lysat de bactéries Vitreoscilla filiformis dans le milieu de fermentation complet a été utilisé sous une forme évapoconcentrée, à des concentrations en extrait sec du lysat de 0.16% et 0.32% ES.

Les effets sur les démangeaisons du cuir chevelu ont été testés et appréciés par comparaison des groupes à J2, J7, J15, J21 et J28 au travers d'évaluations cliniques réalisées selon un protocole conventionnel.

Ces évaluations ont été effectuées par des dermatologues qualifiés selon les techniques habituellement mises en oeuvre dans le domaine.

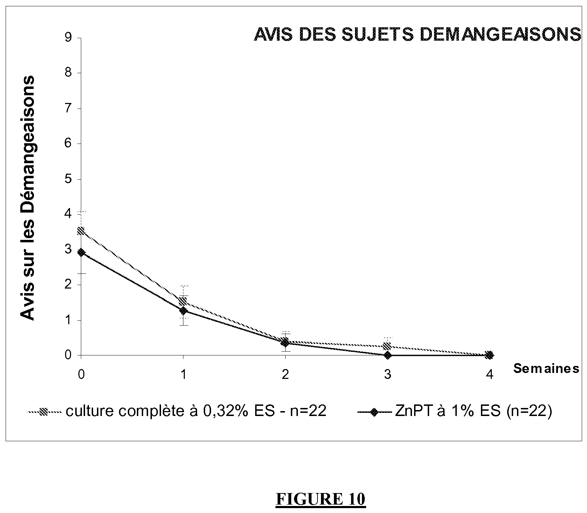

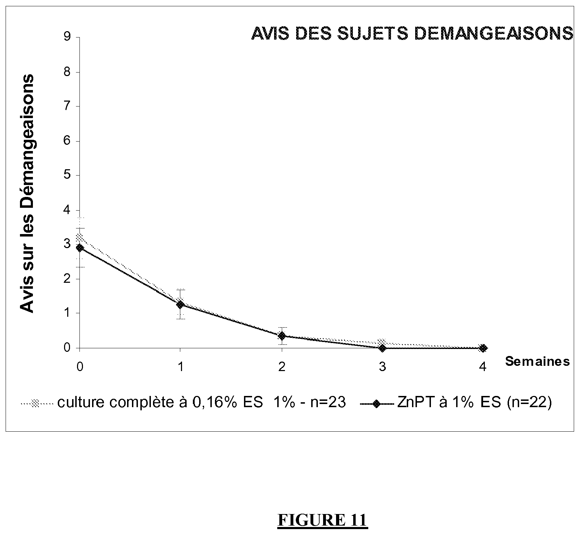

Les résultats sont présentés dans les figures 10 et 11.

La figure 10 illustre les résultats comparatifs d'effets sur les démangeaisons du cuir chevelu (avis des sujets) obtenus en utilisant une formulation de shampoing conforme à l'invention comprenant 0,32% en poids de l'actif (lysat bactérien dans le milieu de fermentation complet), par rapport au poids total de l'extrait sec de la formulation. La formulation comparative de référence comprend 1% en poids d'actif, par rapport au poids total de l'extrait sec.

La figure 11 illustre les résultats comparatifs d'effets sur les démangeaisons du cuir chevelu (avis des sujets) obtenus en utilisant une formulation de shampoing conforme à l'invention comprenant 0,16% en poids de l'actif (lysat bactérien dans le milieu de fermentation complet), par rapport au poids total de l'extrait sec de la formulation. La formulation comparative de référence comprend 1% en poids d'actif, par rapport au poids total de l'extrait sec.

## Revendications

1. Lysat de bactérie(s) appartenant au genre *Vitreoscilla sp.* (notamment espèce: *Vitreoscilla filiformis*), dans un milieu de fermentation complet, à titre d'actif pour son utilisation pour prévenir et/ou traiter les désordres esthétiques liés à des états pelliculaires du cuir chevelu, notamment associés à une prolifération de microorganismes pathogènes sur le cuir chevelu et/ou un déséquilibre de l'écoflore du cuir chevelu,
ledit milieu de fermentation complet étant un milieu issu du procédé de culture ayant été utilisé pour la croissance et la lyse cellulaire du microorganisme dédié à former le dit lysat, ledit milieu n'ayant subi par ailleurs aucune manipulation additionnelle visant à séparer et/ou éliminer tout ou partie de ses constituants non aqueux.

2. Lysat de bactérie(s) appartenant au genre *Vitreoscilla sp.,* dans un milieu de fermentation complet, à titre d'actif pour son utilisation pour prévenir et/ou traiter les désordres esthétiques liés à des états pelliculaires du cuir chevelu, selon la revendication précédente, **caractérisé en ce que** le mélange lysat et milieu de fermentation complet contient les fractions cytoplasmiques, les fragments de paroi cellulaire et les métabolites formés et/ou libérés lors de la lyse cellulaire de ladite bactérie et les métabolites hydrosolubles générées et libérées spontanément par la bactérie lors de son processus de fermentation.

3. Lysat de bactérie(s) appartenant au genre *Vitreoscilla sp.,* dans un milieu de fermentation complet, à titre d'actif pour son utilisation pour prévenir et/ou traiter les désordres esthétiques liés à des états pelliculaires du cuir chevelu, selon l'une quelconque des revendications précédentes, **caractérisé en ce que** ladite bactérie est la souche *Vitreoscilla filiformis.*

4. Lysat de bactérie(s) appartenant au genre *Vitreoscilla sp.,* dans un milieu de fermentation complet, à titre d'actif pour son utilisation pour prévenir et/ou traiter les désordres esthétiques liés à des états pelliculaires du cuir chevelu, selon l'une quelconque des revendications précédentes, **caractérisé en ce que** ladite bactérie est la souche Vitreoscilla filiformis (ATCC 15551).

5. Composition cosmétique et/ou dermatologique comprenant dans un milieu physiologiquement acceptable au moins un lysat de bactérie(s) appartenant au genre *Vitreoscilla sp.* (Notamment espèce: *Vitreoscilla filiformis* ou *Vitreoscilla beggiatoe*), dans leur milieu de fermentation complet, ledit lysat dans le milieu de fermentation complet étant compris dans ladite composition cosmétique en une quantité allant de 0,001% à 10% en poids, par rapport au poids total d'extrait sec de ladite composition,
et ledit milieu de fermentation complet étant un milieu issu du procédé de culture ayant été utilisé pour la croissance et la lyse cellulaire du microorganisme dédié à former le dit lysat, ledit milieu n'ayant subi par ailleurs aucune manipulation additionnelle visant à séparer et/ou éliminer tout ou partie de ses constituants non aqueux.

6. Composition selon la revendication 5, se présentant sous la forme d'une lotion capillaire, d'un shampoing ou d'un après-shampoing.

7. Lysat de bactérie(s) appartenant au genre *Vitreoscilla sp.,* dans un milieu de fermentation complet, à titre d'actif pour son utilisation pour prévenir et/ou traiter les désordres esthétiques liés à des états pelliculaires du cuir chevelu, selon l'une des revendications 1 à 4, **caractérisé en ce qu'**il est administrable par voie topique.

## Patentansprüche

1. Lysat von Bakterie(n), der Gattung Vitreoscilla sp. (insbesondere der Spezies: Vitreoscilla filiformis) zugehörig, in einem Fermentations-Komplettmedium, als Wirkstoff zur Verwendung bei der Prävention und/oder Behandlung von ästhetischen Störungen, die mit Hautzuständen der Kopfhaut verbunden sind, insbesondere mit einer Proliferation von pathogenen Mikroorganismen auf der Kopfhaut und/oder einem Ungleichgewicht der Ökoflora der Kopfhaut einhergehen,
wobei das Fermentations-Komplettmedium ein Medium aus dem Kulturverfahren ist, das für das Zellwachstum und die -lyse des betreffenden Mikroorganismus zum Bilden des Lysats verwendet wurde, wobei das Medium darüber hinaus keinerlei zusätzliche Manipulation durchlaufen hat, die auf die Trennung und/oder Beseitigung von allen oder einem Teil seiner nichtwässrigen Bestandteile ausgerichtet ist.

2. Lysat von Bakterie(n), der Gattung Vitreoscilla sp. zugehörig, in einem Fermentations-Komplettmedium, als Wirkstoff zur Verwendung bei der Prävention und/oder Behandlung von ästhetischen Störungen, die mit Hautzuständen der Kopfhaut verbunden sind, nach dem vorangehenden Anspruch, **dadurch gekennzeichnet, dass** das Lysatgemisch und Fermentations-Komplettmedium Cytoplasmafraktionen, Zellwandfragmente und Metaboliten, die bei der Zelllyse der Bakterie gebildet und/oder freigesetzt werden, und wasserlösliche Metaboliten, die spontan durch die Bakterie bei ihrem Fermentationsprozess erzeugt und freigesetzt werden, enthalten.

3. Lysat von Bakterie(n), der Gattung Vitreoscilla sp. zugehörig, in einem Fermentations-Komplettmedium, als Wirkstoff zur Verwendung bei der Prävention und/oder Behandlung von ästhetischen Störungen, die mit Hautzuständen der Kopfhaut verbunden sind, nach einem der vorangehenden Ansprüche, **dadurch gekennzeichnet, dass** die Bakterie der Stamm Vitreoscilla filiformis ist.

4. Lysat von Bakterie(n), der Gattung Vitreoscilla sp. zugehörig, in einem Fermentations-Komplettmedium, als Wirkstoff zur Verwendung bei der Prävention und/oder Behandlung von ästhetischen Störungen, die mit Hautzuständen der Kopfhaut verbunden sind, nach einem der vorangehenden Ansprüche, **dadurch gekennzeichnet, dass** die Bakterie der Stamm Vitreoscilla filiformis (ATCC 15551) ist.

5. Kosmetische und/oder dermatologische Zusammensetzung, umfassend, in einem physiologisch verträglichen Medium, mindestens ein Lysat von Bakterie(n), der Gattung Vitreoscilla sp. (insbesondere der Spezies: Vitreoscilla filiformis oder Vitreoscilla beggiatoe) zugehörig, in ihrem Fermentations-Komplettmedium, wobei das Lysat in dem Fermentations-Komplettmedium in der kosmetischen Zusammensetzung in einer Menge von 0,001 bis 10 Gew.-% bezüglich des Gesamtgewichts von Trockenextrakt der Zusammensetzung vorhanden ist,
und das Fermentations-Komplettmedium ein Medium aus dem Kulturverfahren ist, das für das Zellwachstum und die -lyse des betreffenden Mikroorganismus zum Bilden des Lysats verwendet wurde, wobei das Medium darüber hinaus keinerlei zusätzliche Manipulation durchlaufen hat, die auf die Trennung und/oder Beseitigung von allen oder einem Teil seiner nichtwässrigen Bestandteile ausgerichtet ist.

6. Zusammensetzung nach Anspruch 5, das in der Form einer Haarlotion, einer Schamponierung oder einer Nachschamponierung dargereicht wird.

7. Lysat von Bakterie(n), der Gattung Vitreoscilla sp. zugehörig, in einem Fermentations-Komplettmedium, als Wirkstoff zur Verwendung bei der Prävention und/oder Behandlung von ästhetischen Störungen, die mit Hautzuständen der Kopfhaut verbunden sind, nach einem der Ansprüche 1 bis 4, **dadurch gekennzeichnet, dass** es topisch verabreichbar ist.

## Claims

1. Lysate of a bacterium or bacteria belonging to the genus *Vitreoscilla sp.* (in particular the species *Vitreoscilla filiformis*) in a complete fermentation medium as active agent for use thereof to prevent and/or treat cosmetic unsightliness related to dandruff conditions of the scalp, in particular associated with proliferation of pathogenic microorganisms on the scalp and/or imbalance of the ecoflora of the scalp;
said complete fermentation medium being a medium derived from the culture method used for growth and cell lysis of the microorganism dedicated to forming said lysate, said medium not having undergone any additional handling intended to separate and/or remove all or part of the non-aqueous constituents thereof.

2. The lysate of a bacterium or bacteria belonging to the genus *Vitreoscilla sp.* in a complete fermentation medium as active agent for use thereof to prevent and/or treat cosmetic unsightliness related to dandruff conditions of the scalp, according to the preceding claim, **characterized in that** the mixture of lysate and complete fermentation medium contains the cytoplasmic fractions, cell wall fragments and metabolites formed and/or released at the time of cell lysis of said bacterium and the water-soluble metabolites spontaneously generated and released by the bacterium during the fermentation process thereof.

3. The lysate of a bacterium and or bacteria belonging to the genus *Vitreoscilla sp.* in a complete fermentation medium as active agent for use thereof to prevent and/or treat cosmetic unsightliness related to dandruff conditions of the scalp, according to any of the preceding claims, **characterized in that** said bacterium is the strain *Vitreoscilla filiformis.*

4. The lysate of a bacterium or bacteria belonging to the genus *Vitreoscilla sp.* in a complete fermentation medium as active agent for use thereof to prevent and/or treat cosmetic unsightliness related to dandruff conditions of the scalp, according to any of the preceding claims, **characterized in that** said bacterium is the strain Vitreoscilla filiformis (ATCC 15551).

5. Cosmetic and/or dermatological composition comprising in a physiologically acceptable medium at least one lysate of a bacterium or bacteria belonging to the genus *Vitreoscilla sp.* (in particular the species: *Vitreoscilla filiformis* or *Vitreoscilla beggiatoe)* in their complete fermentation medium, said lysate in the complete fermentation medium being included in said cosmetic composition in an amount ranging from 0.001% to 10 % by weight, relative to the total weight of dry extract of said composition,
and said complete fermentation medium being a medium derived from the culture method used for growth and cell lysis of the microorganism dedicated to forming said lysate, said medium not having undergone any additional handling intended to separate and/or remove all or part of the non-aqueous constituents thereof.

6. The composition according to claim 5, in the form of a hair lotion, shampoo or conditioner.

7. The lysate of a bacterium or bacteria belonging to the genus *Vitreoscilla sp.* in a complete fermentation medium as active agent for use thereof to prevent and/or treat cosmetic unsightliness related to dandruff conditions of the scalp, according to one of claims 1 to 4, **characterized in that** it can be administered via topical route.
